# EUROPEAN PATENT APPLICATION

(11) **EP 2 016 904 A1**
(43) Date of publication of application: **21.01.2009**
(21) Application number: 07014112.2
(22) Date of filing: 18.07.2007
(51) Int. Cl.: A61B 6/12, A61B 17/22

(54) **Lithotripter**

(71) Applicant: Dornier MedTech Systems GmbH, 82234 Wessling (DE)
(72) Inventor: Buchbauer, Peter, 85748 Garching (DE)
(74) Representative: Grünecker, Kinkeldey, Stockmair & Schwanhäusser Anwaltssozietät

(57) **Abstract**

The invention relates to a lithotripter device (1) having a shock wave source (2) attached to a movable support arm (3) and a patient table (5) supported by a holding structure (4), wherein the shock wave source (2) is movable between at least two positions. To improve this lithotripter so that it can take many different application positions and to improve the operative range of the lithotripter, the design of which is nevertheless space-saving and convenient to operate at the same time, it is suggested that the shock wave source (2) is rotatable about the holding structure (4) of the patient table (5) by means of the support arm (3).

## Description

The present invention relates to a lithotripter device with the features of the preamble of claim 1.

The shock waves provided by a lithotripter are broadly used in today's medicine, for example, for disintegration of concrements in the human body. In using the shock waves provided by the shock wave source of the lithotripter, it is desirable to have a positioning mechanism allowing introducing the shock waves into the human body from different sides and angles.

DE 41 12 148 describes a lithotripter, the shock wave source of which is mounted to a support pillar of a patient table by means of a pivot arm having two swivel joints. The first swivel joint allows the pivot arm, and thus the shock wave source, to rotate about a slanted axis running through the focus of the shock wave source and the second swivel joint allows the pivot arm with the shock wave source to rotate about an axis extending horizontally beneath the slanted pivot arm and runs perpendicular to the longitudinal axis of the support pillar of the patient table.

This design permits an isocentric movement of the shock wave source by rotating it about the first, slanted axis and movement of the shock wave source from one long side of the patient table to the other by rotation about the horizontal axis. However, the range of this latter movement of the shock wave source is rather restricted depending on the design of the support arm. Moreover, over-table positions of the shock wave source cannot be achieved by this design. All in all, the design described in DE 41 12 148 offers only a restricted application range.

It is therefore the object of the invention to improve a lithotripter of the above-mentioned kind such that the shock wave source can take many different application positions, and to improve the operative range of the lithotripter, the design of which is nevertheless space-saving and convenient to operate at the same time.

This object is achieved by a lithotripter having the features of claim 1. By permitting rotation of the shock wave source about a holding structure of the patient table taking different shock wave application positions becomes possible. The shock wave source can be rotated from a position next to one long side of the patient table to an opposite position next to the other long side of the patient table, for example, by rotating it beneath the patient table from one side to the other or by rotating it around one end of the patient table, depending on the location of the holding structure and the range of the rotation angle. Since the origin of the support arm carrying the shock wave source can be rotated about the holding structure of the patient table, it and thus the shock wave source can also easily be moved out of the way, for example, if the space beneath the patient table is needed for different medical devices, e.g. an x-ray source.

Further, the holding structure can be located beneath one half of the patient table. This allows the support arm and thus the shock wave source to rotate about the half of the patient table under which the holding structure is located.

Favourably, the holding structure can comprise a rotatable segment carrying the support arm. Thus, the rotation mechanism can be space-savingly integrated into the holding structure.

Additionally, the support arm can be hinged to the rotatable segment, thereby permitting further mobility of the support arm.

In an embodiment of the invention, the segment can be rotatable about the middle axis of the holding structure, thus allowing a rather uniform rotation of the support arm.

Advantageously, the rotating angle of the rotatable segment can be up to 90° to 360°, particularly up to 100° to 300° and more particularly up to 120° to 270°, thereby permitting a good range of operation for lithotripsy.

Further, the rotatable segment can be located in the lower half, particularly in the lower third and more particularly in the lower fourth of the holding structure being closest to the floor on which the patient table stands. This allows a favourable design of the support arm and permits the support arm to take a relatively low position for rotating beneath the patient table.

In a favourable embodiment of the invention, the support arm is hinged to the holding structure by a joint having one rotational degree of freedom, thereby providing the support arm's mobility with reasonable movement, thus facilitating the use of the lithotripter.

Additionally, the support arm can comprise at least two arm members hinged to each other by a joint having one rotational degree of freedom, thereby forming a compulsory guide for the shock wave source.

In an embodiment of the invention, an arm member of the support arm hinged to the holding structure can be longer than an arm member carrying the shock wave source. This allows a good utilization of space and comfortable positioning of the shock wave source.

Favourably, the ratio of the length of the arm member hinged to the holding structure to the length of the arm member carrying the shock wave source is in a range of 1 to 2.5 and particularly in a range of 1.5 to 2. This allows a good positioning and ergonomic movement of the shock wave source.

Advantageously, the arm member carrying the shock wave source can be capable of moving the shock wave source isocentrically in relation to the focus of the shock wave source, thus forming a compulsory guide for the shock wave source.

Further, the arm members can be movable concentrically to each other, thus allowing one arm member to be pivoted within a sphere given by the other arm member.

The object is further attained with the features of claim 14.

By rotating the support arm and thus the shock wave source about the holding structure of the patient table, the shock wave source can take many application positions and can be easily moved to the desired positions. This allows a broad application range of the lithotripter as well as a more convenient and comfortable operation of the lithotripter.

Additionally, the movable support arm can be rotated about the middle axis of the holding structure, thereby generating a rather uniform rotation of the support arm with the shock wave source.

Further, the shock wave source can be moved from one longitudinal side of the patient table to the other longitudinal side of the patient table by rotating the support arm, e.g. through the space beneath the patient table, thus allowing the shock wave source to be moved quickly and conveniently from one side to the other side of the patient table.

Favourably, the shock wave source can be moved from one longitudinal side of the patient table to the other longitudinal side of the patient table by rotating the support arm about one end of the patient table under which the holding structure is located, thus permitting a quick changing of the shock wave source from one side of the patient table to the other, even if the space under the patient table should already be occupied, for example, by another medical device like an x-ray source.

In an embodiment of the invention, the shock wave source can be moved into a parking position when it is not needed, thereby positioning the shock wave source in a remote area, making way around the patient table, for example, for positioning other medical devices like an x-ray source or for facilitating accessibility of the patient table.

Advantageously, the upper, free arm member of the multi-piece support arm carrying the shock wave source can be moved isocentrically in respect to the focus of the shock wave source, thereby allowing a quick and easy positioning of the shock wave source at different application positions.

In the following, an embodiment of the invention is described based on the following drawings:
- Figure 1: shows a perspective view of an embodiment of the inventive lithotripter with the shock wave source positioned on the left long side of a patient table in an over-table position,
- Figure 2: shows a perspective view of the lithotripter shown in Figure 1 with the shock wave source positioned on the right long side of the patient table in an under-table position and
- Figures 3 a to e: show a motion sequence of the inventive lithotripter shown in Figures 1 and 2.

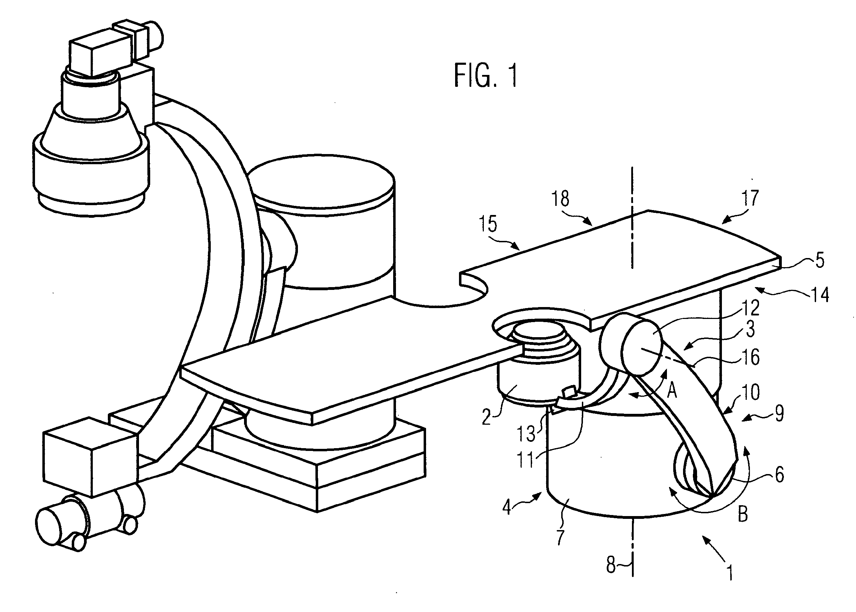

Figure 1 shows a perspective view of an embodiment of the inventive lithotripter 1. The lithotripter 1 has a shock wave source 2 supported by a movable support arm 3. The support arm 3 carrying the shock wave source 2 is hinged to a holding structure 4 of a patient table 5 by a joint 6.

The support arm 3 has multiple arm members. In this embodiment of the invention, the support arm 3 comprises two arm members 10, 11, a lower arm member 10 attached to the holding structure 4 and an upper arm member 11 carrying the shock wave source 2. However, in different embodiments of the invention, the support arm can also comprise more than 2, e.g. 3 or 4, arm members. The upper 11 and the lower arm member 10 are hinged to each other by a joint 12. In this embodiment, the joint 12 has only one rotational degree of freedom, thereby restricting the movement of the arm members 10, 11 relative to each other to the direction indicated by arrow A.

The axis of rotation 16 of the joint 12 extends through a focus point F of the shock wave source 2, thereby forming a compulsory guide for the shock wave source 2. This ensures that the shock wave source 2 moves isocentrically when only the upper arm member 11 is moved.

In this embodiment, both arm members 10, 11 are curved and can be moved by means of joint 12 concentrically to each other. Thus, the upper arm member 11 can be pivoted within the sphere given by the curvature of the lower arm member 10. In an alternative embodiment of the invention, this can also be vice versa, depending on which arm member represents the inner sphere and which one the outer sphere. However, in a different embodiment of the invention, the arm members can also be of a different shape and need not necessarily be curved. Thus, one or both arm members can also be straight or angled.

In this particular embodiment of the invention, the length of the arm members 10, 11 measured along the curved shape of the arm members is different. The lower arm member 10 is longer than the upper arm member 11. Generally, it is favourable when the lower arm member 10 is as long as to allow its outermost edge, namely the joint 12, to be positioned at least at the height of the patient table 5. This allows a good and convenient positioning of the shock wave source 2. Taking the average patient table height into account, basically a length ratio of the lower arm member 10 to the upper arm member 11 of 1 to 2.5 and especially of 1.5 to 2 is advantageous for a good positioning of the shock wave source 2. However, depending on the height of the patient table used with the lithotripter 1 and on the application, in alternative embodiments, different length ratios may also be realized.

The shock wave source 2 is attached to the free end of the upper arm 11 by means of a guide 13.

By means of the support arm 3, the shock wave source 2 is rotatable about the holding structure 4 of the patient table 5. In this particular embodiment of the invention, this rotation mechanism is realized by providing the holding structure 4 with a rotational segment 7 to which the lower arm member 11 of the support arm 3 is attached via joint 6. The rotatable segment 7 of the holding structure 4 is capable of rotating about a middle axis 8 of the holding structure 4 and is arranged in the lower half 9 of the holding structure 4, that is to say in the half 9 closest to the floor on which the patient table stands.

The axis of rotation 8 about which the shock wave source can be rotated by means of the support arm 3 does not necessarily need to be the middle axis of the support structure. It can also be arranged offset of the middle axis. Preferably, the axis of rotation 8 is an approximately vertical axis, which means an axis being essentially perpendicular to the ground on which the holding structure 4 of the patient table 5 stands. If the patient table can be rotated about a vertical axis, it can coincide with the axis of rotation 8 about which the shock wave source with the support arm can be rotated.

In this embodiment of the invention, the holding structure 4 is arranged under one half 18 of the patient table 5, thereby permitting a great mobility range of the shock wave source 2. This allows the shock wave source 2 to be pivoted through the space under the patient table 5 as well as around one end 17 of the patient table, beneath which the holding structure 4 is arranged, in order to change from one long side 14 to the other long side 15 of the patient table 5. The rotatable segment 7 and thus the support arm 3 carrying the shock wave source 2 can be rotated 360 ° about the holding structure 4, thereby allowing a great mobility of the support arm 3. However, in different embodiments of the invention, the holding structure 4 can also be arranged at different positions as, for example, beneath the opposite half of the patient table 5 or even in the middle area of the patient table 5, depending on the application and on other medical devices the lithotripter 1 is used with. In these alternative embodiments of the invention, also the rotational angle of the rotatable segment 7 can be restricted depending on the application such that the rotation angle lies in a range from zero up to 100° to 300 ° or in a range from zero up to 120° to 170°.

In the present embodiment of the invention, the joint 6 has only one rotational degree of freedom, thereby restricting the movement of the lower arm member to a rotation in the direction of arrow B.

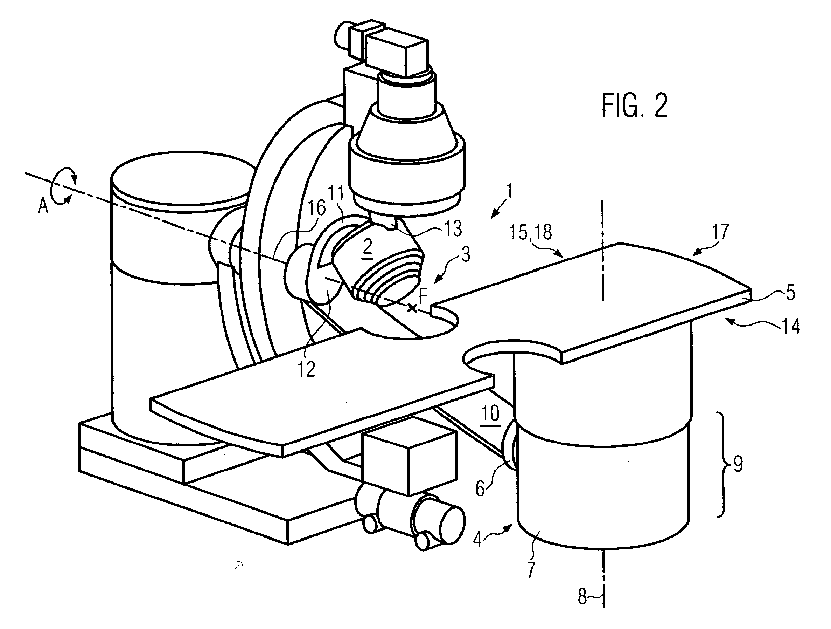

By rotation of the rotatable segment 7, the support arm 3 and the shock wave source 2 can be moved from a position at the right long side 14 of the patient table 5, as shown in Figure 1, to a position at the left long side 15 of the patient table 5, as shown in Figure 2. The shock wave source 2 can also be moved from an under-table position, as shown in Figure 1, to an over-table position, as shown in Figure 2, by rotating the upper arm member 11 of the support arm 3 with respect to the lower arm member 10 about axis 16 by means of joint 12. In Figure 2, the same reference signs are used as in Figure 1, so that in this regard it is referred to the description of Figure 1.

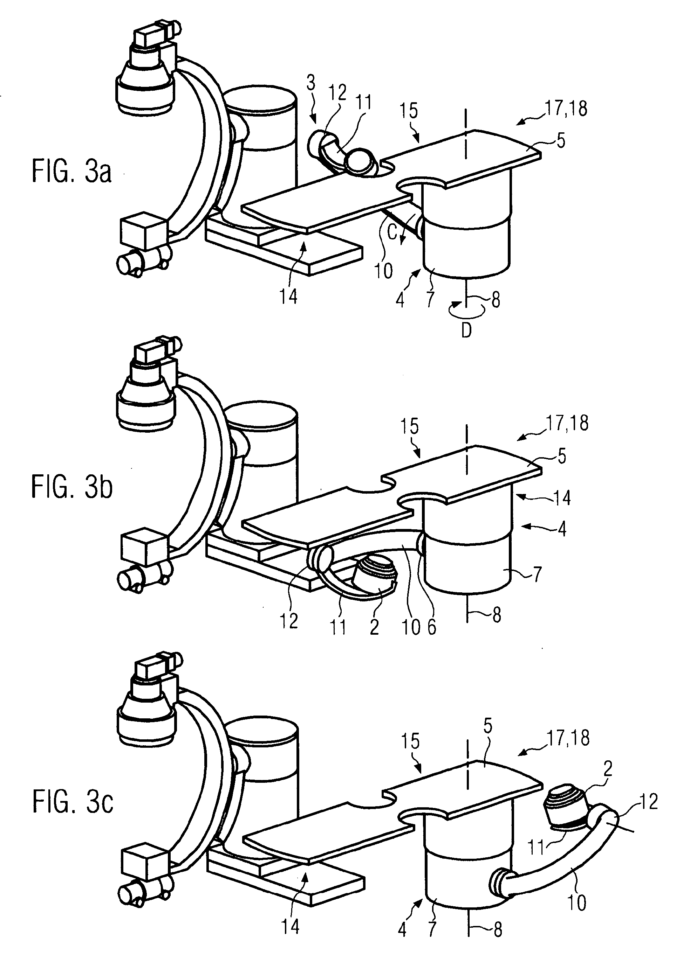

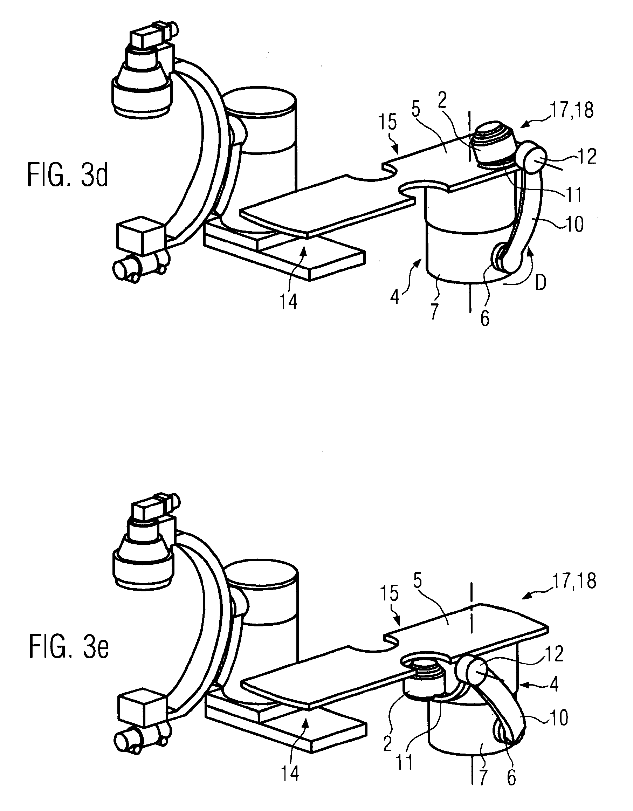

In the following, the mode of operation of the inventive embodiment shown in the figures is described based on the motion sequence shown in Figures 3 a to e. In Figures 3 a to e, the same reference signs are used as in Figures 1 and 2, so that in this regard it is referred to the description of Figures 1 and 2.

In Figure 3a, the shock wave source 2 is positioned next to the left long side 15 of the patient table 5. In order to move the shock wave source 2 into a position next to the right long side 14 of the patient table 5, as shown in Figure 3e, there are basically two opportunities. If there is enough space beneath the patient table 5, the shock wave source 2 can be pivoted through the space under the patient table or, if the space under the patient table is occupied by, for example, an x-ray source, as shown in Figure 2, the shock wave source 2 can be pivoted about one end 17 of the patient table 5.

First, pivoting the shock wave source 2 under the patient table 5 will be described based on Figures 3 a to e. Starting from a left side position shown in Figure 3a, the lower arm member 10 is rotated in the direction of arrow C by means of joint 6 until the height of the outmost end of the lower arm member 10, that is in this case joint 12, is smaller than the height above the floor of the patient table 5.

In this context, care should be taken that the upper arm member 11 and the shock wave source 2 are, by means of joint 12 and guide 13, positioned such that the shock wave source 2 will not hit the floor or any other medical device standing nearby when the whole support arm 3 is rotated about axis 8. When this is ensured, the rotatable segment 7 of the holding structure 4 of the patient table 5 is rotated about axis 8, thereby rotating the support arm 3 through the space under the patient table 5, as shown in Figure 3b.

By further rotating of the rotatable segment 7 and thus of the origin, namely the joint 6 of the support arm 3, the support arm 3 and the shock wave source 2 take a position next to the right long side 14 of the patient table 5, as shown in Figure 3c.

In order to move the shock wave source to an application position, the lower arm member 10 of the support arm 3 can be pivoted by means of joint 6 in the direction of arrow D, as shown in Figure 3d, until the lower arm member 10 reaches its final application position, as shown in Figure 3e. Here, the definite application position of the shock wave source 2, for example, within a recess of the patient table, can be achieved by pivoting the upper arm member 11 by means of joint 12 and moving the shock wave source 2 by means of guide 13.

Alternatively, if the space under the patient table 5 is already occupied, as shown in Figure 2, the support arm 3 with the shock wave source 2 can be rotated about one end 17 of the patient table 5. Starting from Figure 3a with the shock wave source 2 next to the left long side 15 of the patient table 5, the rotatable segment 7 of the holding structure 4 is rotated the other way around, namely against the direction given by arrow D. This results in the support arm 3 rotating about the end 17 of the patient table 5 beneath which the holding structure 4 is located, until the shock wave source 2 takes a position next to the right long side 14 of the patient table 5, as shown in Figure 3c. Then, for achieving a final application position of the shock wave source 2, the same adjustment can be carried out as described above and shown in Figures 3d and 3e.

If the shock wave source 2 is not needed, it can be brought into a parking position. This position is characterized in that the shock wave source 2 takes a remote position in relation to the patient table 5, as shown for example in Figure 3c, thereby making way around the patient table 5 for placing other medical devices like, for example, an x-ray C-bow, next to as well as under the patient table 5.

Although in this particular embodiment of the invention, the rotation mechanism for rotating the support arm 3 carrying the shock wave source 2 is realized by means of a rotational segment of the holding structure 4, this embodiment is not limiting to the invention. It should be clear from this application that the invention and the inventive rotation mechanism can also be achieved by different means, as for example by attaching the support arm 3 to a slide capable of sliding around the holding structure 4 of the patient table 5 on a guide. In this case, the guide could be an integral part of the holding structure 4, or, alternatively, a separate part attached thereto. Also, the guide could extend all around the holding structure 4 permitting 360° rotation of the support arm 3 or extend only partially along the holding structure 4.

## Claims

1. Lithotripter device (1) having a shock wave source (2) attached to a movable support arm (3) and a patient table (5) supported by a holding structure (4), wherein the shock wave source (2) is movable between at least two positions
**characterized in that**
the shock wave source (2) is rotatable about the holding structure (4) of the patient table (5) by means of the support arm (3).

2. Device according to claim 1
**characterized in that**
the holding structure (4) is located beneath one half (18) of the patient table (5).

3. Device according to any of the preceding claims
**characterized in that**
the holding structure (4) comprises a rotatable segment (7) carrying the support arm (3).

4. Device according to any of the preceding claims
**characterized in that**
the support arm (3) is hinged to the rotatable segment (7).

5. Device according to any of the preceding claims
**characterized in that**
the segment (7) is rotatable about the middle axis (8) of the holding structure (4).

6. Device according to any of the preceding claims
**characterized in that**
the rotating angle of the rotatable segment (7) is up to 90° - 360°, particularly up to 100° - 300° and more particularly up to 120° - 270°.

7. Device according to any of the preceding claims
**characterized in that**
the rotatable segment (7) is located in the lower half (9), particularly in the lower third and more particularly the lower fourth of the holding structure (4) being closest to the floor on which the patient table (5) stands.

8. Device according to any of the preceding claims
**characterized in that**
the support arm (3) is hinged to the holding structure (4) by a joint (6) having one rotational degree of freedom.

9. Device according to any of the preceding claims
**characterized in that**
the support arm (3) comprises at least two arm members (10, 11) hinged to each other by a joint (12) having one rotational degree of freedom.

10. Device according to any of the preceding claims
**characterized in that**
an arm member (10) of the support arm (3) hinged to the holding structure (4) is longer than an arm member (11) carrying the shock wave source (2).

11. Device according to any of the preceding claims
**characterized in that**
the ratio of the length of the arm member (10) hinged to the holding structure (4) to the length of the arm member (11) carrying the shock wave source (2) is in a range of 1 - 2,5 and particularly in a range of 1,5 - 2.

12. Device according to any of the preceding claims
**characterized in that**
the arm member (11) carrying the shock wave source (2) is capable of moving the shock wave source (2) isocentrically in relation to the focus (F) of the shock wave source (2).

13. Device according to any of the preceding claims
**characterized in that**
the arm members (10, 11) are movable concentrically to each other.

14. Positioning method for positioning a shock wave source attached to a movable support arm in relation to a patient table supported by a holding structure,
**characterized in that**
the shock wave source (2) is rotated about the holding structure (4) of the patient table (5) by means of the movable support arm (3).

15. Positioning method according to claim 14
**characterized in that**
the movable support arm (3) is rotated about the middle axis (8) of the holding structure (4).

16. Positioning method according to any of the claims 14 - 15
**characterized in that**
the shock wave source (2) is moved from one longitudinal side (14) of the patient table (5) to the other longitudinal side (15) of the patient table (5) by rotating the support arm (3) through the space beneath the patient table (5).

17. Positioning method according to any of the claims 14 - 16
**characterized in that**
the shock wave source (2) is moved from one longitudinal side (14) of the patient table (5) to the other longitudinal side (15) of the patient table (5) by rotating the support arm (3) about one end (17) of the patient table (5) under which the holding structure (4) is located.

18. Positioning method according to any of the claims 14 - 17
**characterized in that**
the shock wave source (2) is moved into a parking position when it is not needed.

19. Positioning method according to any of the claims 14 - 18
**characterized in that**
the upper, free arm member (11) of the multi piece support arm (3) carrying the shock wave source (2) is moved isocentrically in respect to the focus (F) of the shock wave source (2).
